# EUROPEAN PATENT SPECIFICATION

(11) **EP 4 021 402 B1**
(45) Date of publication and mention of the grant of the patent: **20.08.2025**
(21) Application number: 20856392.4
(22) Date of filing: 25.06.2020
(51) Int. Cl.: A61K 9/00, A61K 8/899, A61Q 19/00, A61Q 1/04, A61K 9/06, A61K 31/407, A61K 31/4174, A61K 31/496, A61K 31/573, A61K 47/34, A61K 47/36, A61P 5/44, A61P 31/00

(54) **THIXOTROPIC DELIVERY SYSTEMS**
THIXOTROPE ABGABESYSTEME
SYSTÈMES D'ADMINISTRATION THIXOTROPE

(30) Priority: 26.08.2019 US 201962891631 P
(43) Date of publication of application: 06.07.2022
(73) Proprietor: University of Montana, Missoula MT 59812 (US)
(72) Inventor: SERBAN, Monica, Missoula, Montana 59812 (US)
(74) Representative: Guardian IP Consulting I/S
(86) International application number: PCT/US2020/039551
(87) International publication number: WO 2021/040873

(56) References cited:
- KR-A- 20170 061 870
- US-A1- 2017 290 855
- US-A1- 2019 106 673
- SHEN LULU ET AL: "Self-assembly of silica spheres on silk fibroin spheres for synthesis of porous hollow silica spheres and their in vitro biocompatibility and drug delivery property", JOURNAL OF NON-CRYSTALLINE SOLIDS, NORTH-HOLLAND PHYSICS PUBLISHING. AMSTERDAM, NL, vol. 522, 13 July 2019 (2019-07-13), XP085821998, ISSN: 0022-3093, [retrieved on 20190713], DOI: 10.1016/J.JNONCRYSOL.2019.119557
- RONGHUA JIN ET AL: "Multiple-Responsive Mesoporous Silica Nanoparticles for Highly Accurate Drugs Delivery to Tumor Cells", ACS OMEGA, vol. 3, no. 4, 18 April 2018 (2018-04-18), US, pages 4306 - 4315, XP055737559, ISSN: 2470-1343, DOI: 10.1021/acsomega.8b00427
- SANTOS MOLIRIA V., PECORARO ÉDISON, SANTAGNELI SILVIA H., MOURA ANDRÉ L., CAVICCHIOLI MAURÍCIO, JEREZ VLADIMIR, ROCHA LUCAS A., DE: "Silk fibroin as a biotemplate for hierarchical porous silica monoliths for random laser applications", JOURNAL OF MATERIALS CHEMISTRY C, vol. 6, 17 January 2018 (2018-01-17), pages 2712 - 2723, XP055796450

## Description

### CROSS-REFERENCE TO RELATED APPLICATIONS

This application claims the benefit of U.S. Provisional Application No. 62/891,631, filed August 26, 2019.

### STATEMENT REGARDING FEDERAL FUNDING

This invention was made with government support under R41DC017641 awarded by the National Institutes of Health. The government has certain rights in the invention.

### FIELD

The present disclosure provides compositions and kits for use in the treatment and prevention of an ear infection.

### BACKGROUND

For therapeutic and prophylactic treatments to be successful, the active agents need to be maintained at a certain concentration level for a period of time at the location or locations of interest within a subject or patient. Although treatment for diseases and disorders can be easily controlled in a hospital setting, treatment regimens are increasingly being done at home by the patient or a caregiver, or in locations without access to hospital care, including military installations and remote towns and villages. Patient compliance with the therapeutic and prophylactic treatment regimen becomes a significant challenge when the treatments are moved outside a hospital setting due to a number of factors, including complicated storage and preparation protocols or difficult administration requirements. It is estimated that only about half of patients who leave a physician's office with a prescription for a treatment regimen do so as directed.

The most obvious result of non-compliance is that the disease or disorder may not be relieved or cured, and in some case can actually lead to a worsening of the disease or disorder. For example, missed doses or early cessation of antibiotic or antiviral therapy may lead to resistant organisms. If patients administered the therapeutic and prophylactic treatments as directed many nursing home admissions, hospital admissions, physician visits, diagnostic tests, and other unnecessary treatments could be avoided.

For example, in the United States, according to a comprehensive 5-year report generated by the Centers of Disease Control and Prevention, approximately 2.4 million health care visits are diagnosed with outer ear infections (otitis externa or OE) annually. Both the pediatric and adult population are affected by OE, with a higher prevalence recorded in adults (approximately 53% of cases). The global ear infection treatment market is forecasted to have an incremental growth of $2.79 billion by 2023, with the largest market share being attributed to North America and Europe.

The most common cause accounting for approximately 98% of all ear infections is bacterial septicity typically attributable to *Pseudomonas aeruginosa* or *Staphylococcus aureus.* Treatment of OE involves patient or caregiver administered topical antibiotic drops multiple times daily for 7-14 days. Compliance with the treatment regimen, however, poses significant challenges for the target population, especially for military personnel, school nurses, nursing homes, prisons, the geriatric population, and for patients with head or hand tremors.

Incorrect application or non-compliance with administration schedule of antibiotics often translates to ineffective drug doses at the infection site and leads to infection persistence, recurrence and development of antibiotic resistant bacterial strains. In elderly, diabetic or immunocompromised patients, OE can spread to the surrounding tissue as necrotizing or malignant otitis externa (MOE), causing bone erosion, cranial nerve deficits, abscesses or even death. MOE has been primarily associated with *P. aeruginosa* infections and ciprofloxacin-resistant strains have been found in as many as 33% of isolates. Alarmingly, several recent studies have reported MOE rates to be increasing worldwide. Patients diagnosed with MOE currently undergo more than six weeks of bacteria-culture specific oral systemic antibiotic treatment. Shen et al. (Journal of Non-Crystalline Solids; 522 (2019)) disclose the self-assembly of silica spheres on silk fibroin spheres for synthesis of porous hollow silica spheres and their in vitro biocompatibility and drug delivery property.

Thus, formulations for therapeutic and prophylactic treatment which decrease complicated storage and dosage or administration requirements and increase ease of use are desired for remote and in-home settings.

### SUMMARY

Disclosed herein are compositions for use in treatment or prevention of an ear infection, wherein the composition comprises an antibiotic, a hydrolyzed tetraethyl orthosilicate (TEOS), at least one macromolecule selected from hyaluronan and silk fibroin, and water; wherein the composition has thixotropic properties; and wherein the composition is administered to the ear topically in liquid form or as a **gel.** In some embodiments, the compositions are thixotropic hydrogels.

Also disclosed herein, but not claimed, are methods of manufacturing the compositions comprising obtaining hydrolyzed TEOS, preparing an aqueous solution of the at least one macromolecule in water, wherein the macromolecule is selected from the group consisting of hyaluronan and silk fibroin; and mixing the hydrolyzed TEOS with the aqueous solution of macromolecule. The methods may further comprise adding at least one active agent.

Also disclosed herein are pre-filled delivery devices comprising the composition, kits comprising the composition and a delivery device, according to the claims.

Other aspects and embodiments of the disclosure will be apparent in light of the following detailed description and accompanying figures.

### BRIEF DESCRIPTION OF THE DRAWINGS

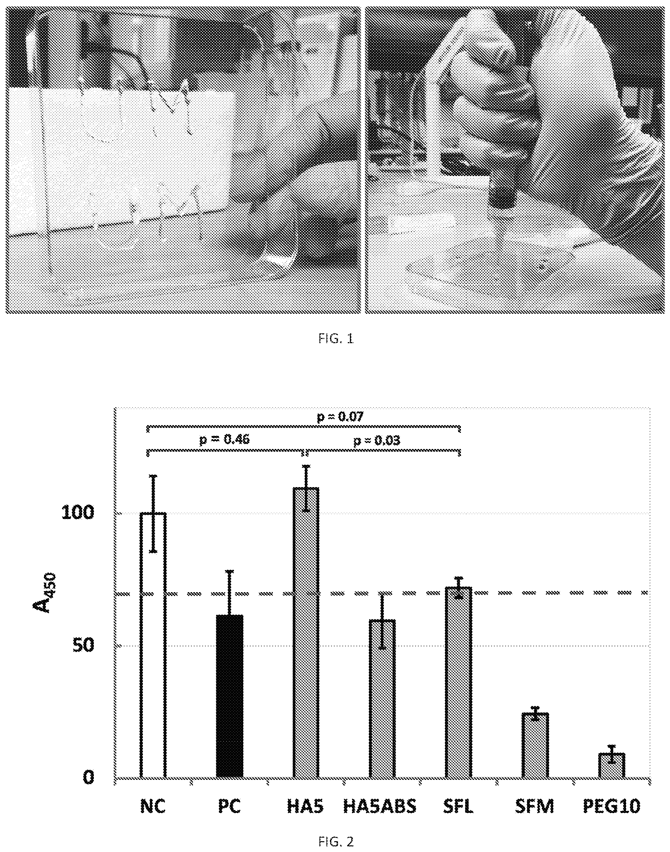

FIG. 1 is images showing the University of Montana initials (UM) (left) drawn by deploying a thixogel though a 26G 1/2 needle (right). The thixogel liquefies when pushed through the needle/syringe nozzle and rapidly gels once deployed.
FIG. 2 is a graph of the cytocompatibility of various thixogel formulations as determined by a colorimetric methyltetrazolium assay (MTT) with primary human dermal fibroblasts. A TEOS-only thixogel was used as positive control (PC), while cells with no treatment were used as negative control (NC). HA5 - hyaluronan MW 5 kDa/TEOS; HA5ABS - chemically modified HA5 with 4-aminobutyl triethoxysilane/TEOS; SFL - silk fibroin low ratio/TEOS; SFL - silk fibroin high ratio/TEOS; PEG10 - polyethylene glycol MW 10 kDalTEOS.

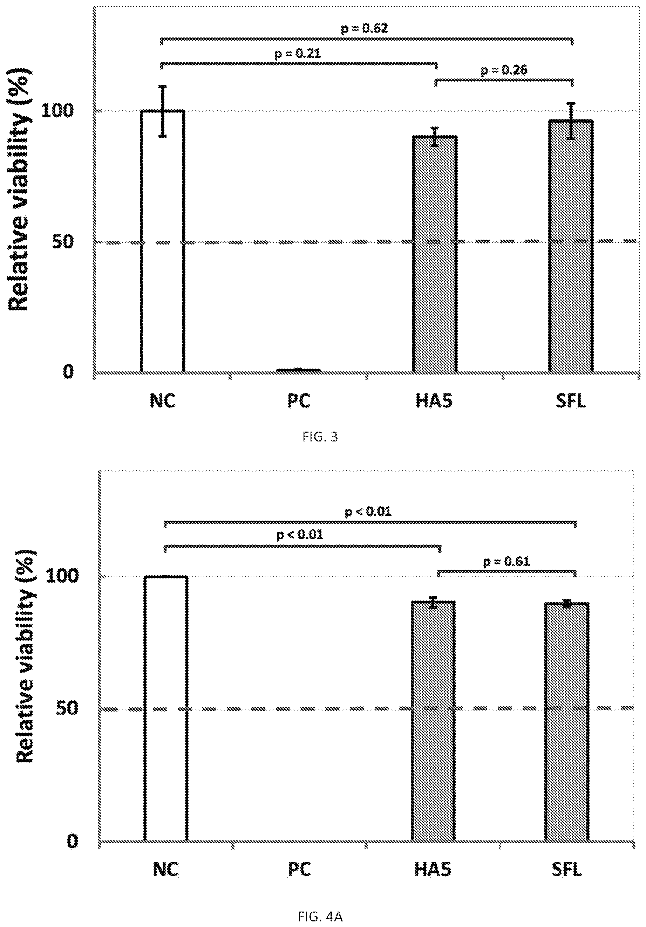

FIG. 3 is a graph of the standardized skin irritation test with EpiDerm^{™} indicating that the THIXOGELS were non-irritant per UN GHS. NC - negative control. An irritant is defined as a test substance that reduces tissue viability ≤ 50% of the mean viability (dotted red line) of the negative controls (NC); NC - Dulbecco's Phosphate Buffered Saline; PC - 5% Sodium Dodecyl Sulfate solution.

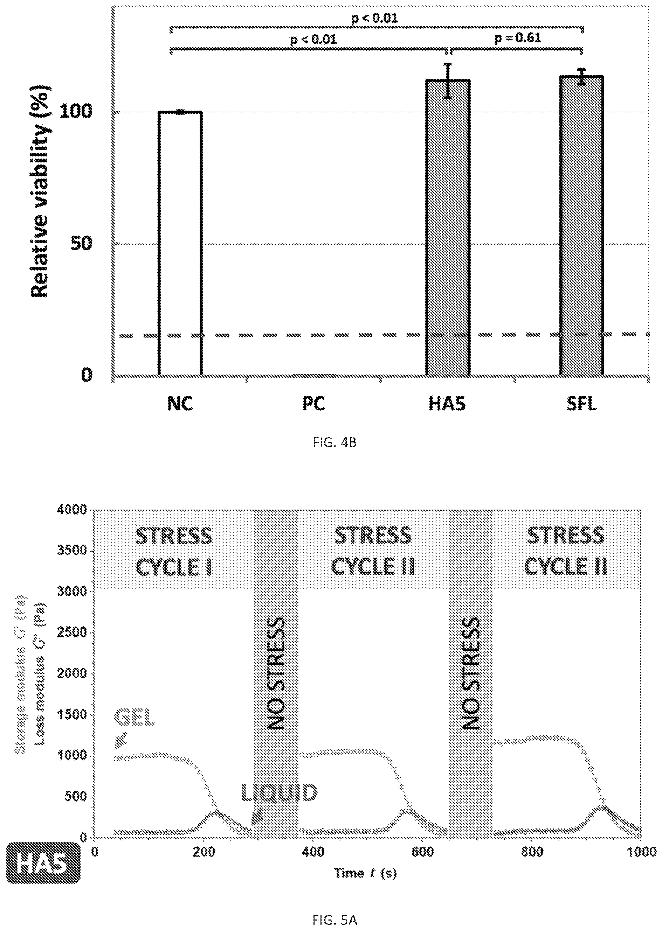

FIGS. 4A and 4B are graphs of skin corrosion testing of THIXOGELS with EpiDerm^{™}. After 3 minutes of exposure to HA5 or SFL formulations, the viability of tissues was above the 50% threshold (dotted line, FIG. 4A); after the 1-hour exposure (FIG. 4B), tissues treated with both formulations fully recovered and had better viability that the negative control (NC) H₂O treated tissues. As positive control (PC) an 8N KOH solution was used.

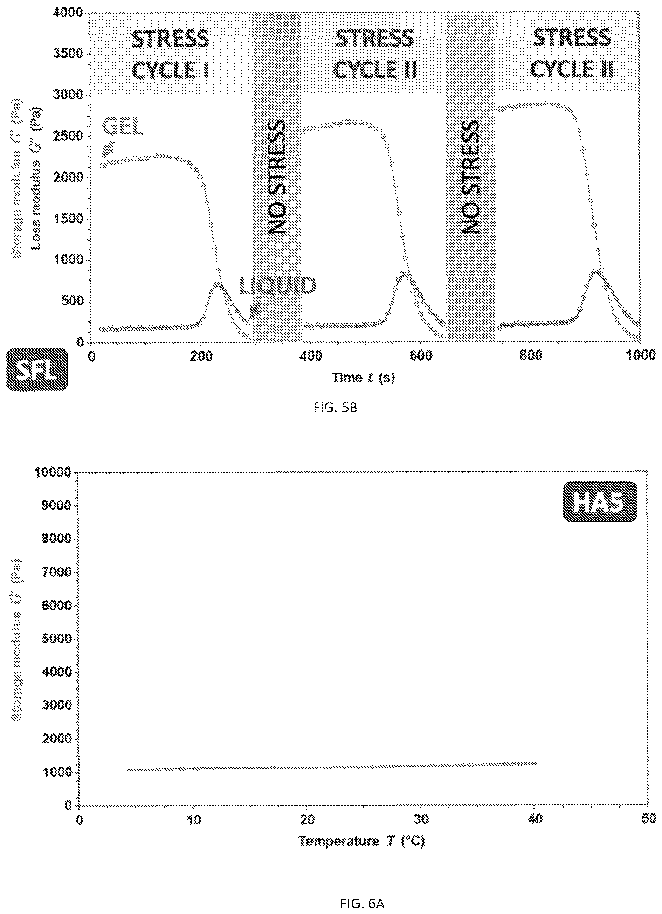

FIGS. 5A and 5B show graphs of representative thixotropy tests for HA5 and SFL thixogels. The graphs show that the materials liquefy [G" (green line) > G' (blue line)] when stressed and re-gel (G' > G") when the stress is removed over the three cycles tested. For both formulations, after the first cycle, G' values were higher, most likely due to polymeric network consolidation through solvent exclusion.

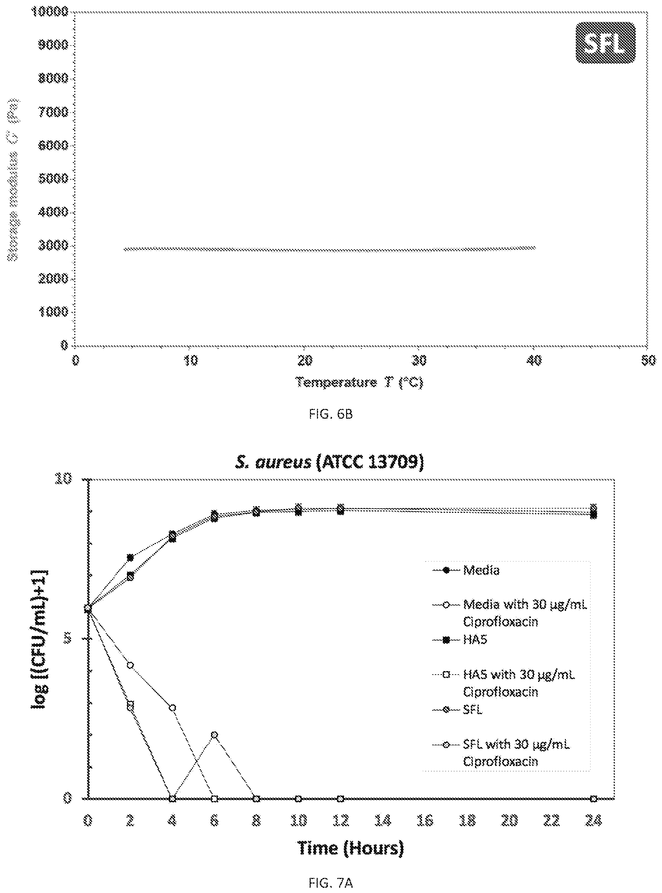

FIGS. 6A and 6B are graphs of representative temperature dependent behavior of thixogels made with HA (FIG. 6A) and SFL (FIG. 6B), indicating a modest increase in material stiffness values at temperatures above 60°C, most likely attributable to water loss.

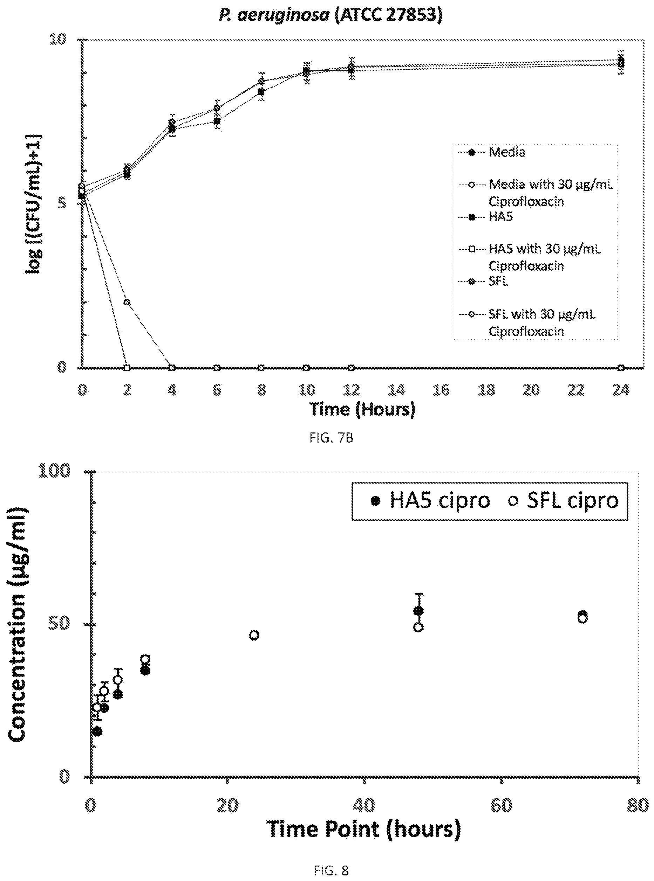

FIGS. 7A and 7B are graphs of the effects of HA and SF gels on the kill kinetics of ciprofloxacin against S. aureus 13709 (FIG. 7A) and P. aeruginosa 27853 (FIG. 7B). The minimum inhibitory concentration (MIC) for ciprofloxacin is 0.25 µg/mL (from Table 2); the 30 µg/mL is a dose that is ~100 times lower than the intended clinical dose of ciprofloxacin (3,000 µg/mL or 0.3% w/v). Growth was measured by sampling media placed above the thixogel and then quantifying colony forming units (CFU's) by serial dilution.
FIG. 8 is a graph of ciprofloxacin release profiles. Both HA and SL gels released the loaded drug at comparable rates via diffusion. The equilibrium concentration between gels and supernatant saline was reached within 24 hours.

### DETAILED DESCRIPTION

The present disclosure provides cyto- and bio-compatible compositions according to the claims comprising a hydrolyzed tetraethyl orthosilicate (TEOS), at least one macromolecule selected from the group consisting of hyaluronan and silk fibroin, and water. In some embodiments, the compositions comprise a hydrolyzed tetraethyl orthosilicate (TEOS), at least one macromolecule selected from the group consisting of hyaluronan and silk fibroin, and a biocompatible buffer. The compositions are thixotropic and exist as gels but liquefy when under shear stress, such as when passed through a syringe nozzle, such that they can be delivered as a liquid or a gel. The compositions allow controlled release of an active agent, thereby dropping the required dosage of the active agent. The observed drug release profile suggests rapid delivery of a therapeutically effective dose and the maintenance of this concentration at the administration site.

The compositions are stable over a broad temperature range including room and physiological temperature, removing the need for temperature dependent handling. These properties overcome many of the challenges which exist with existing gel-based products. For example, Otiprio^{®}, a slow-release formulation based on a thermosensitive liquid-to-gel system that contains 6% ciprofloxacin, was approved by the FDA in March 2018 for the treatment of otitis externa. However, the thermosensitive aspect of this therapeutic requires multiple preparation steps and handling protocols that open the possibility of user error and noncompliance, including: use of multiple components for preparation (first needle for dosing, a catheter for syringe priming, and a second syringe for bilateral applications) and the material needs to be kept cold as inadvertent contact with the physician's hand or prolonged exposure to room temperature would cause the material to gel and affect dosing.

Section headings as used in this section and the entire disclosure herein are merely for organizational purposes and are not intended to be limiting.

### 1. Definitions

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "and" and "the" include plural references unless the context clearly dictates otherwise. The present disclosure also contemplates other embodiments "comprising," "consisting of" and "consisting essentially of," the embodiments or elements presented herein, whether explicitly set forth or not.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated.

Unless otherwise defined herein, scientific and technical terms used in connection with the present disclosure shall have the meanings that are commonly understood by those of ordinary skill in the art. For example, any nomenclatures used in connection with, and techniques of, cell and tissue culture, molecular biology, immunology, microbiology, genetics and protein and nucleic acid chemistry and hybridization described herein are those that are well known and commonly used in the art. The meaning and scope of the terms should be clear; in the event, however of any latent ambiguity, definitions provided herein take precedent over any dictionary or extrinsic definition. Further, unless otherwise required by context, singular terms shall include pluralities and plural terms shall include the singular.

As used herein, the term "preventing" refers to partially or completely delaying onset of an infection, disease, disorder and/or condition; partially or completely delaying onset of one or more symptoms, features, or manifestations of a particular infection, disease, disorder, and/or condition; partially or completely delaying progression from an infection, a particular disease, disorder and/or condition; and/or decreasing the risk of developing pathology associated with the infection, the disease, disorder, and/or condition.

As used herein, "treat," "treating" and the like means a slowing, stopping or reversing of progression of a disease or disorder when provided a composition described herein to an appropriate control subject. The term also means a reversing of the progression of such a disease or disorder to a point of eliminating or greatly reducing the symptoms of the disease or disorder. As such, "treating" means an application or administration of the compositions described herein to a subject, where the subject has a disease or a symptom of a disease, where the purpose is to cure, heal, alleviate, relieve, alter, remedy, ameliorate, improve or affect the disease or symptoms of the disease.

As used herein, the terms "providing", "administering," "introducing," are used interchangeably herein and refer to the placement of the compositions of the disclosure into a subject by a method or route which results in at least partial localization of the composition to a desired site. The compositions can be administered by any appropriate route which results in delivery to a desired location in the subject.

As used herein, the term "thixotropic" refers to a property of a composition, e.g. a hydrogel, that enables it to flow when subjected to a mechanical force such as a shear stress or when agitated and return to a gel-like form when the mechanical force is removed. Thus, "thixotropy" refers to the time and shear rate dependence of viscosity of a fluid.

As used herein, the term "subject" refers to any animal (e.g., a mammal), including, but not limited to, humans and non-human animals. The terms "subject" and "patient" may be used interchangeably herein in reference to a human subject. The term "non-human animals" refers to all non-human animals including, but are not limited to, vertebrates such as rodents, non-human primates, ovines, bovines, ruminants, lagomorphs, porcines, caprines, equines, canines, felines, ayes, etc. The subject may include either adults or juveniles (e.g., children). In one embodiment of the methods and compositions provided herein, the mammal is a human.

A "syringe" is a device comprising a barrel, typically but not necessarily tube-shaped, for injecting/applying or withdrawing a sample in a thin stream, typically through a hollow needle. Samples are injected/applied or withdrawn via pressure, typically from a reciprocating pump (e.g., employing a piston or plunger). A plunger can be linearly pulled and pushed along the inside of the barrel, allowing the syringe to take in and expel liquid or gas through a discharge orifice at the front (open) end of the tube. The open end of the syringe may be fitted with a needle, a nozzle or a tubing to help direct the flow into and out of the barrel. The syringe barrel may be divided into multiple chambers, such that individual components of a composition or sample to be injected mix upon application or injection only.

A "peptide" or "polypeptide" is a linked sequence of two or more amino acids linked by peptide bonds. The peptide or polypeptide can be natural, synthetic, or a modification or combination of natural and synthetic. Polypeptides include proteins such as binding proteins, receptors, and antibodies. The proteins may be modified by the addition of sugars, lipids or other moieties not included in the amino acid chain. The terms "polypeptide" and "protein," are used interchangeably herein.

The term "controlled release" herein refers to any formulation or dosage form that comprises an active drug and which is formulated to release the drug for longer duration of time and to provide a longer duration of pharmacological response after administration of the dosage form than is ordinarily experienced after administration of a corresponding immediate release formulation comprising the same drug in the same amount. Controlled release formulations include, inter alia, those formulations described elsewhere as "prolonged release," "delayed release," "sustained release," "extended release," "programmed release," "time release," and/or "rate controlled" formulations or dosage forms.

The term "hydrogel" herein refers to a specific type of gel in which water-swellable polymeric matrices that can absorb a substantial amount of water in a three-dimensional network of macromolecules held together by covalent or noncovalent crosslinks.

As used herein, the term "fluoroquinolones" refers to a class of antibiotics which exert their antibacterial effects by inhibiting bacterial DNA gyrase and which include a fluorinated quinolone ring system. Exemplary fluoroquinolones include, without limitation, ciprofloxacin, enrofloxacin, enoxacin, gatifloxacin, gemifloxacin, levofloxacin), lomefloxacin, moxifloxacin, norfloxacin, ofloxacin, sparfloxacin, trovafloxacin, difloxacin, cinofloxacin, pefloxacin, tosufloxacin, temafloxacin, fleroxacin, amifloxacin, binfloxacin, danofloxacin, marbofloxacin, ruflocaxin, and sarafloxacin.

Preferred methods and materials are described below, although methods and materials similar or equivalent to those described herein can be used in practice or testing of the present disclosure. The materials, methods, and examples disclosed herein are illustrative only and not intended to be limiting.

### 2. Compositions

Disclosed herein are compositions comprising a hydrolyzed tetraethyl orthosilicate (TEOS), at least one macromolecule and water. In some embodiments, the compositions comprise a hydrolyzed tetraethyl orthosilicate (TEOS), at least one macromolecule and a biocompatible buffer. The biocompatible buffer includes those approved and commonly in use for parenteral delivery of drugs, examples of which can be found in Pharmaceutical Dosage Form: Parenteral Medications, Volume 1, 2^{nd} Edition, Chapter 5, p. 194, De Luca and Boylan, "Formulation of Small Volume Parenterals", Table 5: Commonly used additives in Parenteral Products. The biocompatible buffer may include, but is not limited to, those derived from acetic, ascorbic, aspartic, citric, glutaric, gluconic, lactic, malic, succinic, phosphate, tartaric, and carbonic acids, those using amino acids (e.g. histidine), those including amines (e.g. tromethamine (Tris)), and those used for injectables (e.g. normal saline, bacteriostatic water, bacteriostatic sodium chloride solution, dextrose 5% water, Ringer's solution, lactated Ringer's solution) as known in the art. The at least one macromolecule is selected from the group consisting of hyaluronan and silk fibroin. In some embodiments, the composition is a thixotropic hydrogel.

In some embodiments, the compositions comprise about 0.1% to about 20% (w/v) of the at least one macromolecule, based on volume of hydrolyzed TEOS. The composition may include about 0.1% (w/v) of the at least one macromolecule, about 0.5% (w/v) of the at least one macromolecule, about 1% (w/v) of the at least one macromolecule, about 2% (w/v) of the at least one macromolecule, about 3% (w/v) of the at least one macromolecule, about 4% (w/v) of the at least one macromolecule, about 5% (w/v) of the at least one macromolecule, about 6% (w/v) of the at least one macromolecule, about 7% (w/v) of the at least one macromolecule, about 8% (w/v) of the at least one macromolecule, about 9% (w/v) of the at least one macromolecule, about 10% (w/v) of the at least one macromolecule, about 11% (w/v) of the at least one macromolecule, about 12% (w/v) of the at least one macromolecule, about 13% (w/v) of the at least one macromolecule, about 14% (w/v) of the at least one macromolecule, about 15% (w/v) of the at least one macromolecule, about 16% (w/v) of the at least one macromolecule, about 17% (w/v) of the at least one macromolecule, about 18% (w/v) of the at least one macromolecule, about 19% (w/v) of the at least one macromolecule, or about 20% (w/v) of the at least one macromolecule, based on volume of hydrolyzed TEOS. In select embodiments, the composition comprises about 10% (w/v) of the at least one macromolecule, based on volume of hydrolyzed TEOS. In select embodiments, the composition comprises about 0.5% (w/v) of the at least one macromolecule, based on volume of hydrolyzed TEOS.

The at least one macromolecule may be hyaluronan, also known as hyaluronic acid. In some embodiments, the hyaluronan may have a molecular weight less than about 20 kDa. The hyaluronan may have a molecular weight less than 15 kDa, less than 12 kDa, less than 10 kDa, less than 8 kDa, less than 5 kDa or less than 1 kDa. The hyaluronan may have a molecule weight greater than 1kDa, greater than 5 kDa, greater than 8 kDa, greater than 10 kDa, greater than 12 kDa, or greater than 15 kDa. In select embodiments, the hyaluronan has a molecular weight of about 5 kDa. The composition may comprise about 10% (w/v) hyaluronan, based on volume of hydrolyzed TEOS.

The at least one macromolecule may be silk fibroin. In some embodiments, the composition comprises about 0.5% (w/v) silk fibroin, based on volume of hydrolyzed TEOS.

In some embodiments, the composition comprises water and hydrolyzed TEOS at a volume ratio of about 2:1 to about 1:1. In select embodiments the macromolecule is hyaluronan and the volume ratio is about 2:1. In select embodiments, the macromolecule is silk fibroin and the volume ratio is about 1:1.

### a) Active Agent

The composition according to the claims comprises an antibiotic. The active agent may be a drug, protein, enzyme, hormone, polysaccharide, glycoprotein, oligopeptide, steroid, vitamin, antibody or a combination thereof, with analgesic, anesthetic, antimicrobial, anti-fungal, or anti-inflammatory pharmacologic effects. Examples of such active agents include: Antibiotics such as ofloxacin (0.3%), finafloxacin (0.3%), ciprofloxacin or ciprofloxacin HCl (0.2-6%), gentamycin sulfate (0.2, 0.3%), neomycin sulfate (0.3%, 0.4%, 0.5%, 2.5%), Penicillin G procaine (10,000 IU), polymyxin B sulfate (5,000 IU or 0.05%), kanamycin sulfate (0.5%), amphomycin calcium (0.5%), orbifloxacin (1.0%), enrofloxacin (0.5%), silver sulfadiazine (1.0%), and florfenicol or acetate florfenicol (1.0%, 1.7%); Anti-inflammatories such as dexamethasone (0.1%), hydrocortisone (1%), hydrocortisone aceponate (0.1%), hydrocortisone acetate (0.2%, 0.5%, 1%) mometasone furoate or mometasone furoate monohydrate (0.1%, 0.2%), betamethasone or betamethasone valerate (0.1%), isoflupredone acetate (0.1%), and prednisolone acetate (0.3%, 0.5%); Antifungals such as clotrimazole (1%), miconazole nitrate (1.5%, 2.3%), thiabendazole (4%), posaconazole (0.1%), and terbinafine (1%, 1.5%); Anesthetics such as tetracaine hydrochloride (0.5%), and procaine; and Analgesics such as aspirin, naproxen, ibuprofen, and acetaminophen.

The antimicrobial agent may include antibacterial, antifungal, antiviral, antiprotozoal, and/or antiparasitic agents. According to the claims, the antimicrobial agent is an antibiotic. The antibiotic may be selected from the group consisting of (fluoro)quinolones, carbapenems, aminoglycosides, polypeptide antibiotic, phenicols, and derivatives or combinations thereof.

In some embodiments, the active agent is selected from the group of quinolones and quinolone-based medicaments, for example (fluoro)quinolones (e.g., ofloxacin, ciprofloxacin, levofloxacin, trovafloxacin). Non-limiting examples of quinolones and fluoroquinolones include Cinoxacin, Ciprofloxacin, Enoxacin, Gatifloxacin, Grepafloxacin, Levofloxacin, Lornefloxacin, Moxifloxacin, Nalidixic acid, Norfloxacin, Ofloxacin, Sparfloxacin, Trovafloxacin, Oxolinic acid, Gemifloxacin, and Perfloxacin. The quinolone antibiotic may include first generation quinolones (e.g., cinoxacin, cinobac, nalidixic acid), second generation quinolones (e.g., ciprofloxacin, enoxacin, norfloxacin), third generation quinolones (e.g,balofloxacin, levofloxacin, tosufloxacin) fourth generation quinolones (e.g., clinafloxacin, gatifloxacin, moxifloxacin, trovafloxacin) and/or other quinolones (e.g. prulifloxacin).

**In** some embodiments, the antibiotic is ciprofloxacin. Ciprofloxacin can be present in any pharmaceutically acceptable form, e.g., ciprofloxacin hydrochloride, monohydrate. The composition may comprise less than about 3000 µg/mL ciprofloxacin. The composition may comprise about 10 µg/mL to about 3000 µg/mL ciprofloxacin, about 10 µg/mL to about 2500 µg/mL ciprofloxacin, about 10 µg/mL to about 2000 µg/mL ciprofloxacin, about 10 µg/mL to about 1500 µg/mL ciprofloxacin, about 10 µg/mL to about 1000 µg/mL ciprofloxacin, about 10 µg/mL to about 750 µg/mL ciprofloxacin, about 10 µg/mL to about 500 µg/mL ciprofloxacin, about 10 µg/mL to about 250 µg/mL ciprofloxacin, about 10 µg/mL to about 100 µg/mL ciprofloxacin, about 100 µg/mL to about 3000 µg/mL ciprofloxacin, about 100 µg/mL to about 2500 µg/mL ciprofloxacin, about 100 µg/mL to about 2000 µg/mL ciprofloxacin, about 100 µg/mL to about 1500 µg/mL ciprofloxacin, about 100 µg/mL to about 1000 µg/mL ciprofloxacin, about 100 µg/mL to about 750 µg/mL ciprofloxacin, about 100 µg/mL to about 500 µg/mL ciprofloxacin, about 100 µg/mL to about 250 µg/mL ciprofloxacin, about 250 µg/mL to about 3000 µg/mL ciprofloxacin, about 250 µg/mL to about 2500 µg/mL ciprofloxacin, about 250 µg/mL to about 2000 µg/mL ciprofloxacin, about 250 µg/mL to about 1500 µg/mL ciprofloxacin, about 250 µg/mL to about 1000 µg/mL ciprofloxacin, about 250 µg/mL to about 750 µg/mL ciprofloxacin, about 250 µg/mL to about 500 µg/mL ciprofloxacin, about 500 µg/mL to about 3000 µg/mL ciprofloxacin, about 500 µg/mL to about 2500 µg/mL ciprofloxacin, about 500 µg/mL to about 2000 µg/mL ciprofloxacin, about 500 µg/mL to about 1500 µg/mL ciprofloxacin, about 500 µg/mL to about 1000 µg/mL ciprofloxacin, about 500 µg/mL to about 750 µg/mL ciprofloxacin, about 750 µg/mL to about 3000 µg/mL ciprofloxacin, about 750 µg/mL to about 2500 µg/mL ciprofloxacin, about 750 µg/mL to about 2000 µg/mL ciprofloxacin, about 750 µg/mL to about 1500 µg/mL ciprofloxacin, about 750 µg/mL to about 1000 µg/mL ciprofloxacin, about 1000 µg/mL to about 3000 µg/mL ciprofloxacin, about 1000 µg/mL to about 2500 µg/mL ciprofloxacin, about 1000 µg/mL to about 2000 µg/mL ciprofloxacin, about 1000 µg/mL to about 1500 µg/mL ciprofloxacin, about 1500 µg/mL to about 3000 µg/mL ciprofloxacin, about 1500 µg/mL to about 2500 µg/mL ciprofloxacin, about 1500 µg/mL to about 2000 µg/mL ciprofloxacin, about 2000 µg/mL to about 3000 µg/mL ciprofloxacin, about 2000 µg/mL to about 2500 µg/mL ciprofloxacin, or about 2500 µg/mL to about 3000 µg/mL ciprofloxacin. In some embodiments, the composition comprises about 30 µg/mL to about 3000 µg/mL ciprofloxacin.

Carbapenems are members of the beta lactam class of antibiotics, which kill bacteria by binding to penicillin-binding proteins, thus inhibiting bacterial cell wall synthesis. carbapenems possess the broadest spectrum of activity of the beta lactam class and greatest potency against Gram-positive and Gram-negative bacteria. Carbapenems include, without limitation, imipenem, meropenem, ertapenem, doripenem, panipenem, tebipenem, and biapenem.

**In** some embodiments, the antibiotic is imipenem. The composition may comprise less than about 10,000 µg/mL imipenem. The composition may comprise about 50 µg/mL to about 10,000 µg/mL imipenem, about 100 µg/mL to about 10,000 µg/mL imipenem, about 500 µg/mL to about 10,000 µg/mL imipenem, about 1,000 µg/mL to about 10,000 µg/mL imipenem, about 2,000 µg/mL to about 10,000 µg/mL imipenem, about 3,000 µg/mL to about 10,000 µg/mL imipenem, about 4,000 µg/mL to about 10,000 µg/mL imipenem, about 5,000 µg/mL to about 10,000 µg/mL imipenem, about 6,000 µg/mL to about 10,000 µg/mL imipenem, about 7,000 µg/mL to about 10,000 µg/mL imipenem, about 8,000 µg/mL to about 10,000 µg/mL imipenem, about 9,000 µg/mL to about 10,000 µg/mL imipenem, about 50 µg/mL to about 9,000 µg/mL imipenem, about 100 µg/mL to about 9,000 µg/mL imipenem, about 500 µg/mL to about 9,000 µg/mL imipenem, about 1,000 µg/mL to about 9,000 µg/mL imipenem, about 2,000 µg/mL to about 9,000 µg/mL imipenem, about 3,000 µg/mL to about 9,000 µg/mL imipenem, about 4,000 µg/mL to about 9,000 µg/mL imipenem, about 5,000 µg/mL to about 9,000 µg/mL imipenem, about 6,000 µg/mL to about 9,000 µg/mL imipenem, about 7,000 µg/mL to about 9,000 µg/mL imipenem, about 8,000 µg/mL to about 9,000 µg/mL imipenem, about 50 µg/mL to about 8,000 µg/mL imipenem, about 100 µg/mL to about 8,000 µg/mL imipenem, about 500 µg/mL to about 8,000 µg/mL imipenem, about 1,000 µg/mL to about 8,000 µg/mL imipenem, about 2,000 µg/mL to about 8,000 µg/mL imipenem, about 3,000 µg/mL to about 8,000 µg/mL imipenem, about 4,000 µg/mL to about 8,000 µg/mL imipenem, about 5,000 µg/mL to about 8,000 µg/mL imipenem, about 6,000 µg/mL to about 8,000 µg/mL imipenem, about 7,000 µg/mL to about 8,000 µg/mL imipenem, about 50 µg/mL to about 7,000 µg/mL imipenem, about 100 µg/mL to about 7,000 µg/mL imipenem, about 500 µg/mL to about 7,000 µg/mL imipenem, about 1,000 µg/mL to about 7,000 µg/mL imipenem, about 2,000 µg/mL to about 7,000 µg/mL imipenem, about 3,000 µg/mL to about 7,000 µg/mL imipenem, about 4,000 µg/mL to about 7,000 µg/mL imipenem, about 5,000 µg/mL to about 7,000 µg/mL imipenem, about 6,000 µg/mL to about 7,000 µg/mL imipenem, about 50 µg/mL to about 6,000 µg/mL imipenem, about 100 µg/mL to about 6,000 µg/mL imipenem, about 500 µg/mL to about 6,000 µg/mL imipenem, about 1,000 µg/mL to about 6,000 µg/mL imipenem, about 2,000 µg/mL to about 6,000 µg/mL imipenem, about 3,000 µg/mL to about 6,000 µg/mL imipenem, about 4,000 µg/mL to about 6,000 µg/mL imipenem, about 5,000 µg/mL to about 6,000 µg/mL imipenem, about 50 µg/mL to about 5,000 µg/mL imipenem, about 100 µg/mL to about 5,000 µg/mL imipenem, about 500 µg/mL to about 5,000 µg/mL imipenem, about 1,000 µg/mL to about 5,000 µg/mL imipenem, about 2,000 µg/mL to about 5,000 µg/mL imipenem, about 3,000 µg/mL to about 5,000 µg/mL imipenem, about 4,000 µg/mL to about 5,000 µg/mL imipenem, about 50 µg/mL to about 4,000 µg/mL imipenem, about 100 µg/mL to about 4,000 µg/mL imipenem, about 500 µg/mL to about 4,000 µg/mL imipenem, about 1,000 µg/mL to about 4,000 µg/mL imipenem, about 2,000 µg/mL to about 4,000 µg/mL imipenem, about 3,000 µg/mL to about 4,000 µg/mL imipenem, about 50 µg/mL to about 3,000 µg/mL imipenem, about 100 µg/mL to about 3,000 µg/mL imipenem, about 500 µg/mL to about 3,000 µg/mL imipenem, about 1,000 µg/mL to about 3,000 µg/mL imipenem, about 2,000 µg/mL to about 3,000 µg/mL imipenem, about 50 µg/mL to about 2,000 µg/mL imipenem, about 100 µg/mL to about 2,000 µg/mL imipenem, about 500 µg/mL to about 2,000 µg/mL imipenem, about 1,000 µg/mL to about 2,000 µg/mL imipenem, about 50 µg/mL to about 1,000 µg/mL imipenem, about 100 µg/mL to about 1,000 µg/mL imipenem, about 500 µg/mL to about 1,000 µg/mL imipenem, about 50 µg/mL to about 500 µg/mL imipenem, about 100 µg/mL to about 500 µg/mL imipenem, or about 50 µg/mL to about 100 µg/mL imipenem. In some embodiments, the composition comprises about 100 µg/mL to about 10,000 µg/mL imipenem.

Aminoglycosides are a class of antibiotics derived at least part from a saccharide or polysaccharide and having the empirical formula CmHoNpOq (where m, n, p, and q are appropriate integers). For instance, the aminoglycosides are oligosaccharides consisting of an aminocyclohexanol moiety glycosidically linked to other amino sugars. Aminoglycosides include, without limitation, amikacin, apramycin, arbekacin, bambermycins, butirosin, dibekacin, dihydrostreptomycin, fortimicin(s), fradiomycin, gentamicin, ispamicin, kanamycin, micronomicin, neomycin, neomycin undecylenate, netilmicin, paromomycin, ribostamycin, sisomicin, spectinomycin, streptomycin, streptonicozid, and tobramycin.

Polypeptide antibiotics are a chemically diverse class of antibiotics containing non-protein polypeptide chains. The polypeptide antibiotics may be from either of the two classes: non-ribosomally synthesized peptides and ribosomally synthesized (natural) peptides. Polypeptide antibiotics include, without limitation, actinomycin, bacitracins, and polymyxins (e.g. colistin and polymyxin B), Bleomycin, gramicidins, and glycopeptides.

Phenicols are derived from dichloroacetic acid with two other parts: an aromatic nucleus with an alkyl group in the para position and an aminopropanediol chain. Phenicols block peptide elongation by binding to the peptidyltansferase centre of the 70S ribosome. Phenicols include, without limitation, chloramphenicol, thiamphenicol, and florfenicol.

The composition may further comprise at least one pharmaceutically acceptable excipient including carriers, adjuvants, such as preserving, stabilizing, wetting or emulsifying agents, solution promoters, salts for regulating the osmotic pressure, and/or buffers. In those embodiments in which the compositions comprise a biocompatible buffer, an additional buffer is unlikely to be included in the composition, unless such a combination buffer is known in art, such a citrate-histidine. Such carriers, adjuvants, and other excipients will be compatible with the environment in the hydrogel compositions.

### b) Methods of Making

Also disclosed herein, but not claimed, are methods of manufacturing the compositions described herein. The methods may comprise obtaining hydrolyzed TEOS, preparing an aqueous solution of the at least one macromolecule in water, and mixing the hydrolyzed TEOS with the aqueous solution of macromolecule. Hydrolyzed TEOS may be obtained by incubating TEOS under acidic conditions. In some embodiments, TEOS is incubated with acetic acid to yield hydrolyzed TEOS.

The hydrolyzed TEOS and the aqueous solution of macromolecule is mixed at a volume ratio which allows gel formation. In some embodiments, the volume ratio between about 1:1 and about 2:1. The macromolecule is selected from the group consisting of hyaluronan and silk fibroin. In some embodiments, the macromolecule is hyaluronan and the volume ratio is about 2:1. In some embodiments, the macromolecule is silk fibroin and the volume ratio is about 1:1.

The method may further comprise adding at least one active agent. In some embodiments, the at least one active agent is added to the hydrolyzed TEOS or the aqueous solution of the at least one macromolecule. In some embodiments, the at least one active agent is added to the composition following mixing the hydrolyzed TEOS with the aqueous solution of macromolecule.

Descriptions of the macromolecule, active agents, and relative amounts thereof, set forth above in connection with the inventive composition are also applicable to the method of manufacturing the compositions.

### 3. Methods of Use and Delivery

### a) Prefilled delivery device

The disclosure also provides a pre-filled delivery device comprising the compositions described herein. Descriptions of the macromolecules, active agents, and relative amounts thereof, set forth above in connection with the inventive composition are also applicable to the method of manufacturing the compositions.

A pre-filled delivery device is a device which is filled with the composition prior to distribution to the end user that administers the composition. In some embodiments, the drug delivery device has separate chambers for the storage for the hydrolyzed TEOS and the macromolecule. In this embodiment, the hydrolyzed TEOS and the macromolecule are mixed just prior or concomitantly with administration.

The drug delivery device may include any device configured to allow administration or delivery of a liquid or gel composition. The drug delivery device may include, without limitation, an autoinjector, a pen, a dermal patch, an eye/ear dropper, a dropper bottle, a syringe, a pump, a wound dressing (e.g., gauze or bandage), or a transdermal patch or implant.

In some embodiments, the device is a syringe. A pre-filled syringe typically includes a containment container forming part of a syringe body, a plunger, and either an attached hypodermic needle or such features to allow a needle to be attached by the user prior to administration. In some embodiments, the device is a wound or incision dressing.

The device may contain a single dose of the composition or multiple doses which can be measured out and administered over time.

### b) Compositions for use in a method of treating or preventing a disease or disorder

The claims provide a composition- or a kit for use in the method of treatment or prevention of an ear infection. The methods comprise administering an effective amount of the composition described herein to a subject in need thereof. In some embodiments, the composition is administered in a liquid form. In some embodiments, the composition is administered as a gel. Descriptions of the macromolecules, active agents, and relative amounts thereof, set forth above in connection with the inventive composition are also applicable to the method of manufacturing the compositions.

The disease or disorder may comprise infection, inflammation, pain, irritation, loss of tissue or tissue damage, or a combination thereof. The disease or disorder is an ear infection.

The route and regimen of administration will vary depending upon the nature and location of the disease or disorder and is to be determined by the skilled practitioner. The claims demand administration to the ear topically in liquid form or as a gel.

The subject may be a human, a non-human primate, a member of the family Canidae, a member of the family Felidae, a member of the family Equidae, a member of the family Leporid, a member of the family Bovidae, a member of the family Suidae, a member of the family Cervidae, a member of the family Macropodidae, or a member of the family Ursidae.

An "effective amount" of the compositions disclosed herein is an amount that is delivered to a subject, either in a single dose or as part of a series, which is effective for treating or preventing the disease or disorder. The specific dose level may depend upon a variety of factors including the activity of the peptide, composition or vaccine, the age, body weight, general health, and diet of the subject, time of administration, and route of administration.

### c) Method for the delivery of at least one active agent

The composition of the invention may suitably be used for the delivery of at least one active agent, where controlled release of active agent is desired. Provided herein (not claimed) are methods for the delivery of at least one active agent. The methods comprise providing at least one active agent in a thixotropic hydrogel comprising a hydrolyzed tetraethyl orthosilicate (TEOS), at least one macromolecule selected from the group consisting of hyaluronan and silk fibroin, and water.

The active agent may be a drug, protein, enzyme, hormone, polysaccharide, glycoprotein, oligopeptide, steroid, analgesic, anesthetic, vitamin, antimicrobial agent, anti-inflammatory agent, antibody or a combination thereof. The claims demand the presence of an antibiotic.

The antimicrobial agent may include antibacterial, antifungal, antiviral, antiprotozoal, and/or antiparasitic agents. According to the claims, the antimicrobial agent is an antibiotic. The antibiotic may be selected from the group consisting of (fluoro)quinolones, carbapenems, aminoglycosides, polypeptide antibiotic, phenicols, and derivatives or combinations thereof.

Descriptions of the macromolecules, active agents, and relative amounts thereof, set forth above in connection with the inventive composition are also applicable to the compositions for use in a method for the delivery of at least one active agent.

### 4. Kits

Also within the scope of the present disclosure are kits that include compositions as claimed. In some embodiments, the kits comprise the composition described herein and a delivery device. The drug delivery device may include any device configured to allow administration or delivery of a liquid or gel composition. The drug delivery device may include, without limitation, an autoinjector, a pen, a dermal patch, an eye/ear dropper, a dropper bottle, a syringe, a pump, a wound dressing, or a transdermal patch or implant. Descriptions of delivery devices set forth above is also applicable to the kits.

Individual member components of the kits may be physically packaged together or separately. The components of the kit may be provided in bulk packages (e.g., multi-use packages) or single-use packages. The kits can also comprise instructions for using the components of the kit. The instructions are relevant materials or methodologies pertaining to the kit. The materials may include any combination of the following: background information, list of components and their availability information (purchase information, etc.), brief or detailed protocols for using the compositions, troubleshooting, references, technical support, and any other related documents. Instructions can be supplied with the kit or as a separate member component, either as a paper form or an electronic form which may be supplied on computer readable memory device or downloaded from an internet website, or as recorded presentation.

It is understood that the disclosed kits can be employed in connection with the disclosed methods. The kit may further contain additional containers or devices for use with the methods disclosed herein. The kits optionally may provide additional components such wound dressings (gauze, adhesive bandages and the like), cotton swabs or wipes, and cleaning or antibiotic wipes.

The kits provided herein are in suitable packaging. Suitable packaging includes, but is not limited to, vials, bottles, jars, flexible packaging, and the like.

### 5. Examples (It is noted that the claims demand the presence of an antibiotic. Any examples lacking this feature are for reference)

### Example 1

### Methods

*Gel Formation.* TEOS was hydrolyzed with 0.15 M Acetic Acid (HOAc) for 1.5 hours at a 1:9 (v/v/) ratio. Hydrolyzed TEOS (hTEOS) was then combined with HA5 solution (10% w/v) or SF solution (0.5%), respectively in different ratios. The solutions were vortexed and the pH was adjusted to ~2 with 3.0 N HOAc. After 3 hours, the pH was raised to 8.5 with 1.5 N NH₄OH. All solutions formed gels when left unstirred overnight at room temperature. The antibiotic agents were added (as solid or liquid) to the TEOS or macromolecule solution and dissolved/mixed in prior to gel formation, or alternatively, was added as a liquid directly to the gel, followed homogenization by vortexing, up and down pipetting or shaking.

*Rheological characterization.* All hydrogels were characterized within the materials pseudolinear viscoelastic range with a 1.00 mm gap, at 20°C unless otherwise specified. Oscillatory strain sweeps for thixotropy investigation were conducted with 8 mm parallel plate fixtures within a strain range of 1-250% and an angular frequency of 10 rad/sec. The wait time between the cycles was 30 sec. For temperature dependent hydrogel behavior evaluation, samples were loaded onto the 20 mm parallel plate fixtures (to provide a larger temperature exchange surface), equilibrated to 4°C then subjected to a temperature ramp of 5°C/min up to 40°C, at a stain rate of 0.4% and an angular frequency of 10 rad/sec. Swelling tests were performed with an active force adjustment protocol by loading the hydrogels onto the 8 mm fixtures, equilibrating them at 37°C and pre-loading them dry with a 0.1N axial compression force 120 seconds, then adding PBS and monitoring the gap distance for a total of 30 min (1800 sec).

*Cell assays.* Primary adult normal dermal fibroblasts (HDFa) (PCS-201-012, ATCC, Manassas, VA) were used to assess the cytocompatibility of the thixogels. Cells were seeded in a 96-well plate at a density of 10⁵ cells/well in 100 µl in serum-free fibroblast conditioned media (ATCC, Manassas, VA) and inbubated for 24 hours. Subsequently, the media was removed and replaced with 50 µl of thixogel (different formulations) and 50 µl of fresh media followed by incubation for 24 h at 37°C/5% CO₂. Cellular viability was quantified with a Cell-Titer 96 Aqueous One Solution Cell Proliferation Assay (Promega, Madison, WI) and detected via absorbance at 450 nm with a microplate reader.

### Example 2

### Tetraethyl orthosilicate (TEOS) hydrogels with reduced cytotoxicity

In *in vitro* assays, tetraethyl orthosilicate (TEOS) hydrogels loaded with gentamycin effectively inhibited the growth of *P. aeruginosa* and *S. aureus.* These materials exhibited thixotropy which enabled them to liquefy under shear stress but gel once the stress was removed (FIG. 1). However, these TEOS-only based materials were found to induce cellular vacuolation and reduced metabolic activity/viability in primary human dermal fibroblasts and were not readily compatible with biomedical applications. Two thixotropic formulations with macromolecular components (hyaluronan and silk fibroin) were developed.

By hydrolyzing TEOS under acidic conditions and mixing it in different ratios with water or aqueous solutions of macromolecules or large molecular weight polymers, such as polyethylene glycol, hyaluronic acid and silk fibroin, optically transparent hydrogels that liquefy under shear stress or agitation but become gels under static conditions were formed. Mechanistically, it is believed the gels form through the polymerization of a low amount of silicate intermediate obtained by TEOS hydrolysis and concomitant entrapment of the macromolecule/large molecular weight polymer within the polymerized TEOS network.

Since thixogels based solely on aqueous TEOS were cytotoxic to primary cells, several thixogel formulations (HA5 - hyaluronan MW 5 kDa/TEOS; HA5ABS - chemically modified HA5 with 4-aminobutyl triethoxysilane/TEOS; SFL - silk fibroin low ratio/TEOS; SFL - silk fibroin high ratio/TEOS; PEG10 - polyethylene glycol MW 10 kDa/TEOS) were screened for their effect on primary human dermal fibroblast (FIG. 2). Per ISO 10993-5, a material that reduces cell viability to <70% of the negative control (NC) has cytotoxic potential. The results indicate that hyaluronan/TEOS (HA5) and silk fibroin/TEOS (SFL) were cytocompatible formulations, with HA5 being statistically equivalent (TTEST: HA5 versus NC, p = 0.46; SFL versus NC, p = 0.07; HA versus SFL, p = 0.03) to the negative control (NC, non-treated cells). Polyethylene glycol (molecular weight up to 10 kDa), and SFM did not appear to confer the needed level of cytoprotection.

Formulations with alternative silanes, including N,N'-bis-[(3-triethoxysilylpropyl)aminocarbonyl]polyethylene oxide (7-10 EO), bis(3-triethoxysilylpropyl)polyethylene oxide (25-30 EO), N-((6)-aminohexyl)aminomethyl triethoxysilane, and 4-aminobutyltriethoxysilane yielded gels that were not thixotropic. 4-Aminobutyl triethoxysilane (ABS) or N-((6)-aminohexyl)aminomethyl triethoxysilane (AHAM) used by themselves or chemically conjugated to HA did not yield cytocompatible gels.

### Example 3

### Tetraethyl orthosilicate (TEOS)-macromolecule hydrogels are non-irritant and non-corrosive

The outer ear canal and ear drum are lined with epithelial tissue similar to skin. EpiDerm^{™}, a validated and regulatory body accepted in vitro skin model representative of in vivo outcomes of chemical, pharmaceutical and skin care testing, was used to assess the biocompatibility of HA5 and SFL thixogels. EpiDerm^{™} consists of normal human-derived epidermal keratinocytes cultured to form organized basal, spinous and granular layers, and a multilayered stratum corneum containing intracellular lamellar lipid layers arranged in patterns analogous to those found in the human epidermis. Two separate material biocompatibility aspects were evaluated: skin irritability and skin corrosion.

For skin irritability testing, a standardized protocol (OECD TG 439) was used in which pre-conditioned EpiDerm^{™} human skin samples (surface area of 0.63 cm²) were treated with 30 µL test substance for 60 min, followed by removal of test substance and tissue washing. Subsequently, the tissue viability was assessed with MTT after a 42-hour post-incubation period. Skin irritation is defined by the United Nations Globally Harmonized System of Classification and Labeling of Chemicals (UN GHS) as the production of reversible damage to the skin following the application of a test substance. A test substance was predicted to have skin irritation potential if the mean relative tissue viability of three individual tissues exposed to it was reduced below 50% of the mean viability of the negative controls (NC). The results indicate that both HA5 and SFL are non-irritant (FIG. 3), with tissue samples treated with both HA5 and SFL having mean viability values statistically equivalent to the mean viability of NC (TTEST: HA5 versus NC, p = 0.21; SFL versus NC, p = 0.62; HA5 versus SFL, p = 0.26).

Similarly, for skin corrosion assessments HA5 and SFL thixogels were tested according to standardized protocol OECD TG 431. Skin corrosion is defined by the UN GHS as the production of irreversible tissue damage in the skin following the application of a test material. The test included treating the EpiDerm^{™} tissue samples with 50 µl of test material for 3 minutes and 1 hour, respectively. A material was classified as definitely corrosive if the relative tissue viability after 3 minutes treatment with a test material was decreased below 50%. However, materials classified non-corrosive after the 3-minute treatment (viability ≥ 50%) will be re-classified as corrosive if the relative tissue viability after the 1-hour treatment with the test material was decreased below 15%. For the 3-minute exposure, HA5 and SFL treated tissues showed a slight decrease in mean sample viability compared to the NC (TTEST: HA5 and SFL, respectively, versus NC, p < 0.01), but there was no statistical difference between the mean viability of samples treated with HA5 or SFL (TTEST: HA5 versus SFL p = 0.61) (FIG. 4A). After 1-hour exposure, tissues treated with both HA5 and SFL fully recovered (TTEST: HA5 and SFL, respectively, versus NC, p < 0.01), and there was no statistical difference between the mean viability of samples treated with HA5 or SFL (TTEST: HA5 versus SFL, p = 0.61) (FIG. 4B). Overall, the results indicated that after 3-minute and 1-hour treatments the thixogels were non-corrosive.

### Example 4

### Physical properties of tetraethyl orthosilicate (TEOS)-macromolecule hydrogels

For biological evaluations the thixogels were subjected to syringe driven sterile filtration. To reproduce the final material properties as accurately as possible, the thixotropic behavior of sterile filtered HA5 and SFL formulations was investigated rheologically with a Discovery HR-2 hybrid rheometer/dynamic mechanical analyzer (TA Instruments). Both HA5 and SFL thixogels transitioned between gel-sol states as a function of stress over the three cycles tested (FIGS. 5A-5B). The storage moduli values (G') of the HA5 formulation reflective of the gels' stiffness values were in the 0.99 ± 0.32 kPa range. The G' values for SFL were in the 2.40 ± 0.68 kPa range indicating that this formulation produced stiffer materials than HA5; however, both thixogels fell under the category of soft materials. The data clearly indicated that both formulations would deploy as liquids and would gel within seconds once placed in the desired location. For example, if used within the ear, the softness of the materials would likely translate to low interference with sound waves and not interfere with normal hearing while in place. It was previously shown that thixogels revert to a very small amount of dry material within 7 days under physiological conditions. Thus, when applied to an ear, the resulting dry material would most likely get naturally eliminated naturally by the ear, with earwax.

Swelling is a major adverse event typically associated with hydrogels. The thixogels were rheologically tested by measuring the gap change between rheometer fixtures in response to swelling. Over the course of the 30 minutes while immersed in phosphate buffered saline at 37°C (conditions mimicking the ear environment), the volumes of the thixogels increased by 0.23 ± 0.19% for HA5 and by 1.72 ± 0.43% for SFL. These results indicate that the swelling of both thixogels was negligible and showed that a product based on either of these formulations would not cause swelling related adverse effects.

The temperature dependence of these materials was tested to better understand the behavior of the thixogels at potential storage and shipment conditions (refrigeration at 4°C/39.2°F, room temperature 22°C/71.6°F), physiological conditions (37°C/98.6°F) and slightly higher temperatures that might occur during typical shipping/transportation (40°C/104°F). Both formulations tested maintained their viscoelastic behavior (stay as gels) in the tested temperature range (FIGS. 6A-6B), only showing modest changes in the storage moduli compared to the room temperature values (up to 7% decrease in G' at low temperatures and 8.5% increase at high temperatures for HA5; up to 4.7% increase in G' for SFL, see Table 1). The thixogels were stable in gel form in the application-specific temperature range.

**Table 1: Temperature dependent behavior of HA5 and SFL thixogels - relative changes in storage moduli in comparison to the room temperature values**

| ***Thixogel*** | ***HA5*** | | | ***SFL*** | | |
|---|---|---|---|---|---|---|
| *Temperature (°C)* | **4** | **22** | **40** | **4** | **22** | **40** |
| *Storage modulus G' (Pa)* | 1072.73 | 1155.24 | 1238.94 | 2899.69 | 2868.36 | 3052.42 |
| | 675.51 | 712.41 | 759.88 | 2905.38 | 2837.56 | 2955.79 |
| | 836.76 | 911.52 | 1015.2 | 2586.76 | 2653.24 | 2743.20 |
| *Average* | 861.67 | 926.39 | 1004.67 | 2797.28 | 2786.39 | 2917.14 |
| *STDEV* | 199.78 | 221.79 | 239.70 | 182.33 | 116.33 | 158.19 |
| ***Change in G' (%)*** | **-6.99** | **0.00** | **+8.45** | **+0.39** | **0.00** | **+4.69** |

These cyto- and biocompatible thixogels showed negligible swelling under physiological conditions and were stable as gels in the 4°C/39.2°F - 40°C/104°F range.

### Example 5

### Tetraethyl orthosilicate (TEOS)-macromolecule hydrogels for antibiotic delivery

Clinically used antibiotics from different classes such as vancomycin (glycopeptide), ciprofloxacin (fluoroquinolone), imipenem (carbapenem) and gentamycin (aminoglycoside) are potent and effective compounds. The Minimum Inhibitory Concentration (MIC) defines the lowest antibiotic concentration that inhibits 99.9% of bacterial growth. The MIC concentrations of vancomycin, ciprofloxacin, imipenem and gentamycin (Table 2) were measured when the antibiotic was delivered in a thixogel.

Gentamycin was found to be undesirable as both gels tested (SF and HA) have a deleterious effect upon potency. Vancomycin was also found to be undesirable as it did not give coverage of both *S. aureus* (Gram positive) and *P. aeruginosa* (Gram negative) organisms. Imipenem and ciprofloxacin, however, showed potency against both pathogenic bacteria and neither of the gels had a significant impact upon potency. These tests determined the MIC concentration, whereas the customary dosing of ciprofloxacin (3,000 µg/mL) and imipenem (10,000 µg/mL) in eye and ear drops is 12,000 and 5,000 times higher than the MIC concentrations, respectively.

**Table 2. Effect of the thixogel formulation upon the Minimum Inhibitory Concentrations (MIC) of antibiotics against the main two bacterial pathogens associated with otitis externa.**

| | **Minimum Inhibitory Concentrations (MIC) (µg/mL)** | | | | | |
|---|---|---|---|---|---|---|
| | ***S. aureus* 13709** | | | ***P. aeruginosa* 27853** | | |
| | **No Gel** | **SF** | **HA** | **No Gel** | **SF** | **HA** |
| **Vancomycin** | 2 | 4 | 16 | > 4 | > 32 | > 32 |
| **Ciprofloxacin** | 0.25 | 0.125 | 0.125 | 0.25 | 0.25 | 0.125 |
| **Imipenem** | 0.032 | 0.125 | < 0.032 | 2 | > 4 | 4 |
| **Gentamycin** | 1 | 8 | 32 | 2 | 32 | 32 |

To understand how the thixogel might affect the rate at which a loaded antibiotic affects S. *aureus* and *P. aeruginosa* growth, the kill kinetics of ciprofloxacin was measured, with or without HA and SF gels. Ciprofloxacin is most common active pharmaceutical ingredient in topical ear drops. Growth rates in media only, and in media above HA and SF gels were identical and reached cell densities of 10⁹ CFU/mL within twelve hours, as expected. The gels alone did not inhibit bacterial growth. HA and SF gel-loaded antibiotic containing ciprofloxacin concentrations 100 times lower than the 0.3% found in commercial otic solutions (30 µg/mL or ~0.003% w/v), completely inhibited bacterial growth in around four hours for both bacterial strains tested (FIGS. 7A-7B). The gel loaded drug inhibited bacterial growth for both stains more than 3log₁₀ (99.999 % inhibition), corresponding to bactericidal drug effects. These results validated that antibiotics may be delivered in thixogels.

The mechanism of drug release ciprofloxacin loaded gels covered with saline was assessed for drug release over a period of 72 hours. Most of the drug was released within 24 hours via diffusion (data fitting best matches the Makoid-Banakar mathematical release model) as shown in FIG. 8. This rapid drug release profile is promising as this would rapidly facilitate the delivery of a therapeutically effective dose and the maintenance of this concentration at the infection site.

### Example 6

### Tetraethyl orthosilicate (TEOS)-macromolecule hydrogels for delivery of anti-inflammatory agents

TEOS macromolecule hydrogels may be made as described above for the delivery of an antibiotic except, an anti-inflammatory agent (0.5-5% hydrocortisone) is added to the TEOS or macromolecule solution and dissolved/mixed in prior to gel formation or is added as a liquid directly to the gel, with homogenization by vortexing, up and down pipetting or shaking. Characteristics of the resulting hydrogel including rheology, swelling and temperature dependence may be determined as described in Examples 1 and 4.

### Example 7

### Tetraethyl orthosilicate (TEOS)-macromolecule hydrogels for delivery of anesthetics

TEOS macromolecule hydrogels may be made as described above for the delivery of an antibiotic except, an anesthetic (0.1-1% tetracaine hydrochloride) is added to the TEOS or macromolecule solution and dissolved/mixed in prior to gel formation or is added as a liquid directly to the gel, with homogenization by vortexing, up and down pipetting or shaking. Characteristics of the resulting hydrogel including rheology, swelling and temperature dependence may be determined as described in Examples 1 and 4.

### Example 8

### Tetraethyl orthosilicate (TEOS)-macromolecule hydrogels for delivery of analgesics

TEOS macromolecule hydrogels may be made as described above for the delivery of an antibiotic except, an analgesic (10-200 mg/mL antipyrine) is added to the TEOS or macromolecule solution and dissolved/mixed in prior to gel formation or is added as a liquid directly to the gel, with homogenization by vortexing, up and down pipetting or shaking. Characteristics of the resulting hydrogel including rheology, swelling and temperature dependence may be determined as described in Examples 1 and 4.

### Example 9

### Tetraethyl orthosilicate (TEOS)-macromolecule hydrogels for delivery of antifungals

TEOS macromolecule hydrogels may be made as described above for the delivery of an antibiotic except, an antifungal (1-3% miconazole, 0.5-3% clotrimazole, 2-6% thiabendazole) is added to the TEOS or macromolecule solution and dissolved/mixed in prior to gel formation or is added as a liquid directly to the gel, with homogenization by vortexing, up and down pipetting or shaking. Characteristics of the resulting hydrogel including rheology, swelling and temperature dependence may be determined as described in Examples 1 and 4.

### Example 10

### Tetraethyl orthosilicate (TEOS)-macromolecule hydrogels for delivery of a drug combination

Commonly, antibiotic treatments are combined with other active agents, including anti-inflammatories, antifungals, anesthetics, and/or analgesics. To form a TEOS macromolecule hydrogel comprising more than one active agent, a hydrogel may be made as described above. An antibiotic (ciprofloxacin and imipenem, as described above), an antifungal (1-3% miconazole, 0.5-3% clotrimazole, and/or 2-6% thiabendazole) and an anti-inflammatory agent (0.5-5% hydrocortisone) may be added to either the TEOS or macromolecule solution prior to gel formation. Alternatively, or in addition, some or all of the agents may be added either together or sequentially as a liquid to the gel with homogenization. Characteristics of the resulting hydrogel including rheology, swelling and temperature dependence may be determined as described in Examples 1 and 4.

It is understood that the foregoing detailed description and accompanying examples are merely illustrative and are not to be taken as limitations upon the scope of the disclosure, which is defined solely by the appended claims.

## Claims

1. A composition for use in treatment or prevention of an ear infection, wherein the composition comprises an antibiotic, a hydrolyzed tetraethyl orthosilicate (TEOS), at least one macromolecule selected from hyaluronan and silk fibroin, and water; wherein the composition has thixotropic properties; and wherein the composition is administered to the ear topically in liquid form or as a gel.

2. The composition for use according to claim 1, wherein the composition is deployed as a liquid and will gel once placed in the ear.

3. The composition for use according to claim 1 or 2, wherein the composition provides for controlled release of the antibiotic.

4. The composition for use according to any one of claims 1-3, wherein the antibiotic is a (i) a quinolone or fluoroquinolone antibiotic, or (ii) a carbapenems antibiotic.

5. The composition for use according to claim 4, wherein the quinolone or fluoroquinolone antibiotic is selected from Cinoxacin, Ciprofloxacin, Enoxacin, Gatifloxacin, Grepafloxacin, Levofloxacin, Lomefloxacin, Moxifloxacin, Nalidixic acid, Norfloxacin, Ofloxacin, Sparfloxacin, Trovafloxacin, Oxolinic acid, Gemifloxacin, and Perfloxacin; and wherein the carbapenems antibiotic is selected from imipenem, rneropenern, ertapenem, donpenem, pa:njpenem, tehlpenem, and biapenem.

6. The composition for use according to any one of claims 1-5, wherein the antibiotic is ciprofloxacin or imipenem.

7. The composition for use according to claim 6, wherein the composition comprises less than about 3000 µg/mL ciprofloxacin or less than about 10,000 µg/mL imipenem.

8. The composition for use according to claim 6, wherein the composition comprises 30 µg/mL to 3000 µg/mL ciprofloxacin or 100 µg/mL to 10,000 µg/mL imipenem.

9. The composition for use according to any one of claims 1-8, wherein the composition is administered by a syringe.

10. The composition for use according to any one of claims 1-9, wherein the ear infection is caused by *Pseudomonas aeruginosa* or *Staphylococcus aureus.*

11. The composition for use according to any one of claims 1-10, wherein the subject is a human, a non-human primate, a member of the family Canidae, a member of the family Felidae, a member of the family Equidae, a member of the family Leporid, a member of the family Bovidae, a member of the family Suidae, a member of the family Cervidae, a member of the family Macropodidae, or a member of the family Ursidae.

12. The composition for use according to any one of claims 1-11, wherein the subject is a human.

13. A kit for use in treatment or prevention of an ear infection, comprising:
a) a composition comprising an antibiotic, a hydrolyzed tetraethyl orthosilicate (TEOS), at least one macromolecule selected from hyaluronan and silk fibroin, and water; and
b) a delivery device defined by at least one of the following:
i) pre-filled with the composition,
ii) wherein the hydrolyzed TEOS and the at least one macromolecule are stored in two separate chambers,
iii) is a syringe.

14. The kit for use according to claim 13, wherein the delivery device is a syringe prefilled with the composition, and wherein the hydrolyzed TEOS and the at least one macromolecule are stored in two separate chambers.

15. The kit for use according to claim 13 or 14, wherein the antibiotic is ciprofloxacin or imipenem.

## Patentansprüche

1. Zusammensetzung zur Verwendung bei Behandlung oder Vorbeugung einer Ohrenentzündung, wobei die Zusammensetzung ein Antibiotikum, ein hydrolysiertes Tetraethylorthosilikat (TEOS), mindestens ein Makromolekül, ausgewählt aus Hyaluronan und Seidenfibroin, und Wasser umfasst; wobei die Zusammensetzung thixotrope Eigenschaften aufweist; und wobei die Zusammensetzung dem Ohr topisch in flüssiger Form oder als ein Gel verabreicht wird.

2. Zusammensetzung zur Verwendung nach Anspruch 1, wobei die Zusammensetzung als eine Flüssigkeit eingesetzt wird und nach Platzieren in dem Ohr geliert.

3. Zusammensetzung zur Verwendung nach Anspruch 1 oder 2, wobei die Zusammensetzung eine kontrollierte Freisetzung des Antibiotikums bereitstellt.

4. Zusammensetzung zur Verwendung nach einem der Ansprüche 1-3, wobei das Antibiotikum (i) ein Chinolon- oder Fluorchinolon-Antibiotikum oder (ii) ein Carbapenem-Antibiotikum ist.

5. Zusammensetzung zur Verwendung nach Anspruch 4, wobei das Chinolon- oder Fluorchinolon-Antibiotikum ausgewählt ist aus Cinoxacin, Ciprofloxacin, Enoxacin, Gatifloxacin, Grepafloxacin, Levofloxacin, Lomefloxacin, Moxifloxacin, Nalidixinsäure, Norfloxacin, Ofloxacin, Sparfloxacin, Trovafloxacin, Oxolinsäure, Gemifloxacin und Perfloxacin; und wobei das Carbapenem-Antibiotikum ausgewählt ist aus Imipenem, Renopenem, Ertapenem, Donpenem, Panipenem, Tebipenem und Biapenem.

6. Zusammensetzung zur Verwendung nach einem der Ansprüche 1-5, wobei das Antibiotikum Ciprofloxacin oder Imipenem ist.

7. Zusammensetzung zur Verwendung nach Anspruch 6, wobei die Zusammensetzung weniger als etwa 3000 µg/ml Ciprofloxacin oder weniger als etwa 10.000 µg/ml Imipenem umfasst.

8. Zusammensetzung zur Verwendung nach Anspruch 6, wobei die Zusammensetzung 30 µg/ml bis 3000 µg/ml Ciprofloxacin oder 100 µg/ml bis 10.000 µg/ml Imipenem umfasst.

9. Zusammensetzung zur Verwendung nach einem der Ansprüche 1-8, wobei die Zusammensetzung mit einer Spritze verabreicht wird.

10. Zusammensetzung zur Verwendung nach einem der Ansprüche 1-9, wobei die Ohrenentzündung *durch Pseudomonas aeruginosa* oder *Staphylococcus* aureusverursacht wird.

11. Zusammensetzung zur Verwendung nach einem der Ansprüche 1-10, wobei das Subjekt ein Mensch, ein nicht-menschlicher Primat, ein Mitglied der Familie Canidae, ein Mitglied der Familie Felidae, ein Mitglied der Familie Equidae, ein Mitglied der Familie Leporid, ein Mitglied der Familie Bovidae, ein Mitglied der Familie Suidae, ein Mitglied der Familie Cervidae, ein Mitglied der Familie Macropodidae oder ein Mitglied der Familie Ursidae ist.

12. Zusammensetzung zur Verwendung nach einem der Ansprüche 1-11, wobei das Subjekt ein Mensch ist.

13. Kit zur Verwendung bei Behandlung oder Vorbeugung einer Ohrenentzündung, der Folgendes umfasst:
a) eine Zusammensetzung, die ein Antibiotikum, ein hydrolysiertes Tetraethylorthosilikat (TEOS), mindestens ein Makromolekül, ausgewählt aus Hyaluronan und Seidenfibroin, und Wasser umfasst; und
b) eine Abgabevorrichtung, die durch mindestens eines der folgenden Merkmale definiert ist:
i) mit der Zusammensetzung vorgefüllt,
ii) wobei das hydrolysierte TEOS und das mindestens eine Makromolekül in zwei getrennten Kammern gelagert sind,
iii) eine Spritze ist.

14. Kit zur Verwendung nach Anspruch 13, wobei die Abgabevorrichtung eine mit der Zusammensetzung vorgefüllte Spritze ist und wobei das hydrolysierte TEOS und das mindestens eine Makromolekül in zwei getrennten Kammern gelagert sind.

15. Kit zur Verwendung nach Anspruch 13 oder 14, wobei das Antibiotikum Ciprofloxacin oder Imipenem ist.

## Revendications

1. Composition destinée à être utilisée dans le traitement ou la prévention d'une infection de l'oreille, dans laquelle la composition comprend un antibiotique, un orthosilicate de tétraéthyle (TEOS) hydrolysé, au moins une macromolécule choisie parmi l'hyaluronane et la fibroïne de soie, et de l'eau ; dans laquelle la composition présente des propriétés thixotropes ; et dans laquelle la composition est administrée à l'oreille par voie topique sous forme liquide ou en tant que gel.

2. Composition destinée à être utilisée selon la revendication 1, dans laquelle la composition est déployée sous forme liquide et se gélifiera une fois placée dans l'oreille.

3. Composition destinée à être utilisée selon la revendication 1 ou 2, dans laquelle la composition permet une libération commandée de l'antibiotique.

4. Composition destinée à être utilisée selon l'une quelconque des revendications 1-3, dans laquelle l'antibiotique est (i) un antibiotique quinolone ou fluoroquinolone ou (ii) un antibiotique carbapénème.

5. Composition destinée à être utilisée selon la revendication 4, dans laquelle l'antibiotique quinolone ou fluoroquinolone est choisi parmi : cinoxacine, ciprofloxacine, énoxacine, gatifloxacine, grépafloxacine, lévofloxacine, loméfloxacine, moxifloxacine, acide nalidixique, norfloxacine, ofloxacine, sparfloxacine, trovafloxacine, acide oxolinique, gémifloxacine et perfloxacine ; et dans laquelle l'antibiotique carbapénème est choisi parmi : imipénème, méropénème, ertapénème, doripénème. panipénème, tébipénème et biapénème.

6. Composition destinée à être utilisée selon l'une quelconque des revendications 1-5, dans laquelle l'antibiotique est la ciprofloxacine ou l'imipénème.

7. Composition destinée à être utilisée selon la revendication 6, dans laquelle la composition comprend moins d'environ 3 000 µg/ml de ciprofloxacine ou moins d'environ 10 000 µg/ml d'imipénème.

8. Composition destinée à être utilisée selon la revendication 6, dans laquelle la composition comprend 30 µg/ml à 3 000 µg/ml de ciprofloxacine ou 100 µg/ml à 10 000 µg/m d'imipénème.

9. Composition destinée à être utilisée selon l'une quelconque des revendications 1-8, dans laquelle la composition est administrée par une seringue.

10. Composition destinée à être utilisée selon l'une quelconque des revendications 1-9, dans laquelle l'infection de l'oreille est causée par *Pseudomonas aeruginosa* ou *Staphylococcus aureus.*

11. Composition destinée à être utilisée selon l'une quelconque des revendications 1-10, dans laquelle le sujet est un être humain, un primate non humain, un membre de la famille des Canidae, un membre de la famille des Felidae, un membre de la famille des Equidae, un membre de la famille des Leporidae, un membre de la famille des Bovidae, un membre de la famille des Suidae, un membre de la famille des Cervidae, un membre de la famille des Macropodidae ou un membre de la famille des Ursidae.

12. Composition destinée à être utilisée selon l'une quelconque des revendications 1-11, dans laquelle le sujet est un être humain.

13. Kit destiné à être utilisé dans le traitement ou la prévention d'une infection de l'oreille, comprenant :
a) une composition comprenant un antibiotique, un orthosilicate de tétraéthyle (TEOS) hydrolysé, au moins une macromolécule choisie parmi l'hyaluronane et la fibroïne de soie, et de l'eau ; et
b) un dispositif d'administration défini par au moins un élément parmi ce qui suit :
i) prérempli avec la composition,
ii) dans lequel le TEOS hydrolysé et la au moins une macromolécule sont stockés dans deux chambres séparées,
iii) est une seringue.

14. Kit destiné à être utilisé selon la revendication 13, dans lequel le dispositif d'administration est une seringue préremplie avec la composition, et dans lequel le TEOS hydrolysé et la au moins une macromolécule sont stockés dans deux chambres séparées.

15. Kit destiné à être utilisé selon la revendication 13 ou 14, dans lequel l'antibiotique est la ciprofloxacine ou l'imipénème.
